# EUROPEAN PATENT APPLICATION

(11) **EP 4 533 961 A1**
(43) Date of publication of application: **09.04.2025**
(21) Application number: 23201587.5
(22) Date of filing: 04.10.2023
(51) Int. Cl.: A23L 33/00, A61K 31/702, A61P 31/04

(54) **PREPARATION FOR USE IN THE PREVENTION AND TREATMENT OF A PATHOGENIC ENTEROCOCCUS FAECALIS INFECTION**

(71) Applicant: FrieslandCampina Nederland B.V., 3818 LE Amersfoort (NL)
(72) Inventor: CAO, Linqiu, 6708 WH Wageningen (NL); DELSING, Bernardina Johanna Martina, 6708 WH Wageningen (NL); GROENEVELD, Dirk Andries, 6708 WH Wageningen (NL)
(74) Representative: FrieslandCampina IP Department

(57) **Abstract**

The invention relates to a preparation comprising 3'-galactosyllactose, 4'-galactosyllactose, and/or 6'-galactosyllactose for use in the treatment and prevention of a pathogenic *Enterococcus faecalis* infection in a subject. It was found that these compounds - in contrast to fructooligosaccharides and the human milk oligosaccharides 2'-FL, LNT, and LNnT - are able to reduce the abundance of *Enterococcus faecalis* in the microbiota. By inhibiting (out-)growth of *E*. *faecalis* in the gut, infections in other locations of the body can be prevented.

## Description

The present invention generally relates to the field of pathogenic *Enterococcus faecalis* infections and the treatment and prevention of infectious diseases caused by *Enterococcus faecalis.*

*Enterococcus faecalis* is a Gram-positive, commensal bacterium inhabiting the gastrointestinal tracts of humans. *E. faecalis* is found in healthy human intestines and in human stool (feces). It can cause serious infection outside the human gut.

As opportunistic pathogen, however, *E. faecalis* can cause life-threatening infections, such as endocarditis and sepsis, urinary tract infections, and meningitis, especially in a hospital environment, where the naturally high levels of antibiotic resistance found in *E. faecalis* contribute to its pathogenicity. *E. faecalis* has also been frequently found in reinfected, root canal-treated teeth.

Nosocomial infections - i.e. hospital-acquired infections - are responsible for a large part of all hospital-based complications. The frequency of nosocomial infections is expected to increase in view of the rising number of elderly patients, the increased complexity of invasive surgical operations, therapeutic treatments that affect natural defence against infections, and the increase in antibiotic-resistant microorganisms.

*E. faecalis* is generally resistant to many commonly used antimicrobial agents (H. Singh et al., Chemical Biology & Drug Design 94 (2019) 1721-1739). The resistance is mediated by the presence of multiple genes related to drug resistance in the chromosome or plasmid (S. Panthee et al, Genomics 113 (2021) 1534-1542).

Resistance to vancomycin by *E. faecalis* is becoming more common (S.G. Amyes International Journal of Antimicrobial Agents 29 (2007) S43-S52 *and* P. Courvalin, Clinical Infectious Diseases 42 (2006) S25-S34*)* and causes considerable morbidity and mortality, in particular in patients whose immune system has already been weakened (C.A. DiazGranados et al., Clin. Infect. Dis., 41 (2005) 327-333).

Non-digestible oligosaccharides, including human milk oligosaccharides (HMOs), galacto- and fructooligosaccharides (GOS and FOS), are a group of complex carbohydrates with potential direct and indirect anti-pathogenic functions. When administered orally, these oligosaccharides can strengthen gut health by positive effects on the gut microbiome and stimulation of immune homeostasis.

Studies have shown anti-pathogenic effects of GOS on the adhesion of *Escherichia coli* and *Mannheimia haemolytica* to intestinal and respiratory epithelium, respectively; see K. Shoaf, et al., Infect Immun. 74 (2006) 6920-8 and Y. Cai, et al., Biomaterials 283 (2022) 121461. Additionally, both GOS and FOS have been shown to inhibit the growth and biofilm formation of bacteria such as *Pseudomonas aeruginosa* strain PAO1 (M. Ortega-González, et al., PLoS One 9 (2014) e85772).

The present invention relates to the finding that certain galactooligosaccharides - that is: 3'-galactosyllactose (3'-GL), 4'-galactosyllactose (4'-GL) and 6'-galactosyllactose (6'-GL) - are able to reduce the abundance of *Enterococcus faecalis* in the microbiota; in contrast to FOS and the HMOs 2'-fucosyllactose (2'-FL), lacto-N-tertraose (LNT), and lacto-N-neotetraose (LNnT), which were less able to achieve this effect.

By inhibiting (out-)growth of *E. faecalis* in the gut, infections in other locations of the body can be prevented.

The invention therefore relates to a preparation comprising 3'-galactosyllactose, 4'-galactosyllactose, and/or 6'-galactosyllactose for use in the prevention and treatment of a pathogenic *Enterococcus faecalis* infection in a subject.

According to one embodiment, the *Enterococcus faecalis* is resistant to antibiotics, more in particular vancomycin resistant.

The term "treatment", in relation to a given disease or disorder, includes, but is not limited to, inhibiting the disease or disorder (e.g. arresting the development of the disease or disorder), relieving the disease or disorder (e.g. causing regression of the disease or disorder); and/or relieving a condition caused by or resulting from the disease or disorder (e.g. relieving, preventing or treating symptoms of the disease or disorder).

As used herein, the term "prevention" in relation to a given disease or disorder means preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

3'-Galactosyllactose (3'-GL) is the trisaccharide Gal-(beta-1,3)-Gal-(beta-1,4)-Glc. 4'-Galactosyllactose (4'-GL) is the trisaccharide Gal-(beta-1,4)-Gal-(beta-1,4)-Glc. 6'-Galactosyllactose (6'-GL) is the trisaccharide Gal-(beta-1,6)-Gal-(beta-1,4)-Glc. 3'-GL, 4'-GL, and/or 6'-GL can be administered in a suitable matrix, or in a nutritional composition. The trisaccharide(s) may for example be part of a mixture of galactooligosaccharides; such mixture being generally referred to as GOS.

The additional presence of other galactooligosaccharides in GOS may beneficially affect the bifidobacteria abundance in the microbiota, thereby improving the production of short chain fatty acids, which in its turn has a further improving effect on the immune system and/or on treatment or prevention of infections, in particular intestinal infections. GOS is a complex mixture of carbohydrates with differing chain length, linkage type, and degree of branching. The basic structure of galactooligosaccharide (GOS) includes a lactose core at the reducing end which is elongated with up to about seven galactose residues (degree of polymerization of 8; DP8). It preferably comprises 40-100 wt%, more preferably 50-90 wt%, and most preferably 60-80 wt% oligosaccharides (DP≥3) on dry weight and may further contain monosaccharides like glucose and galactose, and disaccharides such as lactose, lactulose, and allolactose. The lactose content is generally in the range 0-60 wt%, preferably 0-40 wt%, and most preferably 0-30 wt%; the monosaccharide content is generally in the range 0-10 wt%; based on dry weight.

Commercial GOS preparations are generally produced via a transgalactosylation reaction by enzymatic treatment of lactose with β- galactosidase enzymes (EC.3.2.1.23), yielding a mixture of oligomers with approximately 100 different types structures with varying DP and linkages. Beta-galactosidase is produced in many microorganisms such as *Bacillus circulans, Aspergillus oryzae, Kluyveromyces marxianus, Kluyveromyces fragilis, Sporobolomyces singularis, Lactobacillus fermentum, and Papiliotrema terrestris (Cryptococcus Papiliotrema terrestris).* Beta-galactosidases differ in their three-dimensional structures, resulting in stereo- and regioselective formation of the glycosidic bonds.

After the enzymatic reaction, GOS is isolated and purified using conventional methods, such as nanofiltration and/or sequential simulated moving bed (SSMB). The resulting product is a GOS-containing syrup, which can be dried (e.g. by spray-drying, freeze-drying, or spray-cooling) to form a powder, if so desired.

3'-GL, 4'-GL, and 6'-GL can be synthesized by regioselective protection of lactose to afford a disaccharide acceptor and chemical glycosylation with a galactose donor, as disclosed in CN 114920788.

Alternatively, 3'-GL, 4'-GL, and 6'-GL can be isolated or concentrated from GOS.

For instance, 4'-GL and 6'-GL GOS can be suitably isolated from GOS prepared by using the beta-galactosidase from *Bacillus circulans.* A commercially available source of GOS is Vivinal^{®} GOS from FrieslandCampina. The main trisaccharides in Vivinal^{®} GOS are 4'-GL and 6'-GL. 4'-GL and 6'-GL can be enriched or purified from such GOS, for instance by size exclusion chromatography. Other commercially available types of GOS from which 6'-GL can be isolated or concentrated are Oligomate 55 and 50 from Yakult, Cup Oligo form Nissin Sugar, Bimuno from Clasado, or Purimune from GTC Nutrition.

3'-GL can be suitably isolated from GOS prepared by using the beta-galactosidase from *S. thermophilus.* Particularly suitable is the use of beta-galactosidase from strain CNCM I-1470 and/or CNCM I-1620 in a process as disclosed in example 4 of FR2723960 or example 6 of EP0778885. *S. thermophilus* CNCM I-1620 was deposited under the Budapest Treaty on 23 August 1995 at Collection Nationale de Cultures de Microorganisms van Institute Pasteur, Paris, France by Compagnie Gervais Danone. Strain *S. thermophilus* CNCM I-1620 is also referred to as strain *S. thermophilus* ST065. *S. thermophilus* CNCM I-1470 was deposited under the Budapest Treaty on 25 August 1994 at Collection Nationale de Cultures de Microorganisms van Institute Pasteur, Paris, France by Compagnie Gervais Danone. The composition of this GOS is also described in more detail in LeForestier et. al., Eur. J. Nutr., 48 (2006) 457-464. Both strains have also been published in WO 96/06924. Other commercially available types of GOS from which 3'-GL can be isolated or concentrated are Bimuno from Clasado and Purimune from GTC Nutrition.

The 3'-GL-, 4'-GL-, and/or 6'-GL-containing preparation according to the present invention may also be a GOS that is rich in 3'-GL, 4'-GL, and/or 6'-GL, such as the types of GOS disclosed above or GOS that is enriched in 3'-GL, 4'-GL, and/or 6'-GL by beta-galactosidase.

The GOS preferably contains 3'-GL, 4'-GL, and/or 6'-GL in a concentration of 2.5-25 wt%, more preferably 5-20 wt%, and most preferably 10-15 wt% based on oligosaccharides other than lactose.

The 3'-GL, 4'-GL, and 6'-GL content of GOS can be determined by the fluorescent labeling of the GOS samples and the reference GL standards with 2-aminobenzamide (2-AB), followed by UPLC analysis. The analysis can be executed with Shimadzu UPLC Nexera 30 series or equal, equipped with a LC 30-AD pump, CTO20-AC oven, SIL-30AD Autosampler and SPD-M20A DAD and Fluorescence detector.

The treatment with GOS according to the present invention may involve the treatment of a human adult, child, or infant subject, more in particular infants, young children, hospitalized patients, and subjects suffering from a weakened immune system.

3'-GL, 4'-GL, and/or 6'-GL is preferably administered to the human subject in a daily dosage 3'-GL, 4'-GL, and/or 6'-GL of at least 0.01 grams, more preferably at least 0.02 grams, even more preferably at least 0.04 grams, more preferably at least 0.06 grams, more preferably at least 0.08 grams, even more preferably at least 0.10 grams, more preferably at least 0.5 grams, even more preferably at least 1.0 gram, and most preferably at least 2.0 grams.

The daily 3'-GL, 4'-GL, and/or 6'-GL dosage is preferably at most 15 grams, more preferably at most 10 grams, even more preferably at most 8 grams, and most preferably at most 4 grams.

The preparation for use according to the invention can be administered in various ways - orally or intranasally. The manner of dosage will, amongst others, depend on the age and condition of the subject to be treated.

If administered orally to an adult or older child in order to treat an oral or respiratory infection, it might be useful to gargle for at least 2 seconds, preferably at least 5 seconds, more preferably at least 10 seconds, and most preferably at least 20 seconds prior to swallowing it, thereby enhancing the contact between GOS and oral mucosal tissue.

Intranasal administration of GOS may involve the use of a spray containing GOS.

Oral administration can be performed via a nutritional composition, such as a food product or beverage. Suitable food products can have a liquid, semi-liquid, or solid constituency. Alternatively, the preparation may have the form of a supplement, e.g. as pill, capsule, gummy, syrup, suspension, or dry powder.

Examples of nutritional compositions are milk-based product such as a milk, milkshake, chocolate milk, yoghurt, cream, cheese, pudding, and ice cream; bars, such as nutritional bars, energy bars, snack bars, cereal bars, and bars for diabetics; liquid products, such as nutritional drinks, diet drinks, liquid meal replacers, sports drinks, and other fortified beverages; savory snacks, such as chips, tortillas, puffed and baked snacks, crackers, pretzels, and savory biscuits; bakery products, such as muffins, cakes, and biscuits; sweets such as gummies and candies; and pastas, such as spaghetti.

Oral administration of the preparation to infants may involve administration as a syrup or suspension or as part of an infant formula.

The term "infant formula" as used herein refers to a nutritional composition intended for infants and as defined in Codex Alimentarius, (Codex STAN 72-1981) and Infant Specialties (incl. Food for Special Medical Purpose) as defined in Codex Alimentarius, (Codex STAN 72-1981). It also refers to a foodstuff intended for particular nutritional use by infants during the first months of life and satisfying by itself the nutritional requirements of this category of person (Article 2(c) of the European Commission Directive 2006/141/EC of 22 December 2006 on infant formulae and follow-on formulae). Infant formulas can encompass the starter infant formulas, the follow-up or follow-on formulas, and young child formulas. Generally a starter formula is for infants from birth as breast-milk substitute, and a follow-up or follow-on formula from the 6th month onwards. So, an infant formula may be dedicated for infants of 0 to 6 months, 6 to 12 months, 12-36 months.

It was particularly observed that the preparation according to the invention was able to reduce the *E. faecalis* population in breastfed children. For these subjects, the preparation should have the form of a supplement to breastfeeding.

### EXAMPLE

Two different staring microbiota were used:
- A pool of fecal material from three breastfed infants between 3 and 6 months of age,
- A pool of fecal material from three formula-fed infants between 3 and 6 months of age

The following test compounds were used:
2'-Fucosyllactose (2'-FL), Lacto-N-tetraose (LNT), lacto-N-neotetraose (LNnT), 3'-GL, 4'-GL, 6'-GL, Vivinal^{®} GOS, a GOS enriched in 3'-GL and 6'-GL prepared by the beta-galactosidase originating from *Paenibacillus pabuli* in accordance with EP 3778625 A1 in the name of Good Shusei Co. ("GODO-GOS"), fructo-oligosaccharide (FOS), and a sugar control.

Vivinal^{®} GOS contains 0.64 wt% 6'-GL, 10.1 wt% 4'-GL, and 0.26 wt% 3'-GL; based on oligosaccharides other than lactose.

GODO-GOS contains 12.7 wt% 6'-GL, 1.5 wt% 4'-GL, and 25.2 wt% 3'-GL; based on oligosaccharides other than lactose.

The microbiota form the breastfed infants was cultured in the i-screen in vitro model of intestinal microbiota as disclosed by Schuren, F.H., et al., J. Prob. Health, vol. 7 (2019), issue 2, No. 212, after incubation for 4 hours in SIEM under anaerobic conditions at 37°C, while shaking at 300 rpm.

The incubated microbiota were transferred to microtiter plates. The test compounds (4 mg/ml) were added. After 18 hours of fermentation under anaerobic conditions at 37°C, samples were collected and subjected to DNA analysis and SCFA analysis.

Total DNA was extracted from the samples using an Agowa/PurePrep protocol. To each 150 µl sample, 500 µl zirconium beads (0.1 mm) and 800 µl CD1 solution (DNeasy 96 Powersoil Pro QIAcube HT kit) were added. Cells were disrupted by bead beating twice for 2 min, with cooling on ice in between and afterwards. After centrifugation for 6 min at 3,000 RPM, 350 µl supernatant was mixed with 300 µl Agowa binding buffer and 10 µl Agowa magnetic beads. Samples were further purified using the PurePrep 96 system (Molgen, The Netherlands) with two wash steps and a final elution step in 65 µl.

Libraries for whole-genome sequencing were prepared using the Illumina DNA prep protocol according to the instructions of Illumina (Illumina DNA Prep Reference Guide). DNA concentrations were standardized across samples. After the tagmentation and clean-up steps, PCR-mediated standard indexed i5 and i7 adapters were added and the library was amplified. Next, the libraries were cleaned-up and pooled. Whole-genome sequencing was performed using the Illumina MiSeq sequencer applying MiSeq V3 chemistry. Sequence pre-processing (host filtering) and analysis (mapping, merging of de paired-end reads, classification and normalization) were performed using the MetaPhlAn software package release 3 with the ChocoPhlAn database release 3 (clade-specific marker genes) for taxonomic classification. The classification was performed at the species level. Multi-Dimensional Scaling (MDS) and PERMANOVA analysis were applied to microbiome data transformed using the Wisconsin double standardization.

Surprisingly, it was found that 3'-GL, 4'-GL, 6'GL, Vivinal^{®} GOS, and GODO-GOS showed a strong decrease in *E. faecalis;* in contrast to all other tested compounds including FOS, and the human milk oligosaccharides 2'-FL, LNT, and LNnT.

| Component tested | Relative abundance of *E. faecalis* in the microbiota (%) | Decrease of *E. faecalis* as compared to the untreated control (%) |
|---|---|---|
| Untreated control | 3,89486 | --- |
| Sugar control | 2,73274 | -30 |
| Vivinal^{®} GOS | 1,50304 | -61 |
| GODO-GOS | 1,42358 | -63 |
| 3'-GL | 1,59219 | -59 |
| 4'-GL | 1,85576 | -52 |
| 6'-GL | 1,27263 | -67 |
| 2'-FL | 3,35124 | -14 |
| LNT | 2,56362 | -34 |
| LNnT | 2,92069 | -25 |
| FOS | 3,01782 | -23 |

## Claims

1. Preparation comprising 3'-galactosyllactose, 4'-galactosyllactose, and/or 6'-galactosyllactose for use in the treatment and prevention of a pathogenic *Enterococcus faecalis* infection in a subject.

2. Preparation comprising 3'-galactosyllactose, 4'-galactosyllactose, and/or 6'-galactosyllactose for use in the treatment and prevention of infectious diseases caused by *Enterococcus faecalis.*

3. Preparation for use according to claim 1 or 2 wherein the *Enterococcus faecalis* is resistant to antibiotics.

4. Preparation for use according to claim 3 wherein the *Enterococcus faecalis* is vancomycin resistant.

5. Preparation according to any one of the preceding claims wherein the preparation 3'-galactosyllactose and/or 6'-galactosyllactose, preferably 6'-galactosyllactose.

6. Preparation for use according to any one of the preceding claims wherein the preparation is or contains galacto-oligosaccharide (GOS) enriched in 3'-galactosyllactose, 4'-galactosyllactose, and/or 6'-galactosyllactose, preferably enriched in 3'-galactosyllactose and/or 6'-galactosyllactose, most preferably enriched in 6'-galactosyllactose.

7. Preparation for use according to any one of the preceding claims wherein GOS is administered to a subject orally or nasal.

8. Preparation for use according to any one of the preceding claims wherein the subject is a human subject.

9. Preparation for use according to claim 8 wherein the human subject is an infant or child.

10. Preparation for use according to claim 9 wherein the human subject is an infant receiving breastfeeding.

11. Preparation for use according to claim 8 wherein the subject is human adult.

12. Preparation for use according to any one claim 8-11 wherein the subject suffers from a weakened immune system.
